# EUROPEAN PATENT SPECIFICATION

(11) **EP 1 147 779 B1**
(45) Date of publication and mention of the grant of the patent: **31.05.2006**
(21) Application number: 00830302.6
(22) Date of filing: 20.04.2000
(51) Int. Cl.: A61K 47/48, A61K 31/575, A61P 1/16

(54) **A method for rendering ursodeoxycholic acid in soluble form pharmaceutical compositions**
Verfahren zur Herstellung ursodeoxycholsäure enthaltender pharmazeutischer Zusammensetzungen in flüssiger Form
Méthode pour l'obtention de compositions pharmaceutiques contenant l'acide ursodeoxycholique sous forme soluble

(43) Date of publication of application: 24.10.2001
(73) Proprietor: BIOPROGRESS S.p.A., I-00165 Rome (IT)
(72) Inventor: Olivieri, Aldo, 00165 Roma (IT)
(74) Representative: Tansini, Elio Fabrizio

(56) References cited:
- WO-A-00/04875
- US-A- 5 534 505
- DATABASE CHEMABS [Online] CHEMICAL ABSTRACTS SERVICE, COLUMBUS, OHIO, US; KAWAGISHI, JUICHI: "Inclusion compds of cholic acids and their injections" retrieved from STN Database accession no. 94:103689 XP002148325 -& JP 55 022616 A (TOKYO TANABE CO., LTD., JAPAN) 18 February 1980 (1980-02-18)
- DATABASE CHEMABS [Online] CHEMICAL ABSTRACTS SERVICE, COLUMBUS, OHIO, US; NAKAZAWA, SHINZO ET AL: "Oral aqueous formulations containing bile acids and dextrins" retrieved from STN Database accession no. 108:26970 XP002148326 & JP 62 153220 A (TOKYO TANABE CO., LTD., JAPAN) 8 July 1987 (1987-07-08)

## Description

The present invention relates to a method for rendering ursodeoxycholic acid in soluble form. The present invention further relates to pharmaceutical compositions containing ursodeoxycholic acid in soluble form and to the related use thereof.

Ursodeoxycholic acid (UDCA) is an acid which is physiologically present in human bile, where it represents a small percentage of total biliary acids. UDCA is able to increase, in humans, the solubilising ability of bile with respect to cholesterol, transforming lithogenous bile into non lithogenous bile. Thanks to this characteristic, UDCA is used in clinical practice in preventing the formation of biliary cholesterol stones or in dissolving them, if they have already been formed.

Ursodeoxycholic acid is a practically insoluble substance. Until now, it has been used only for the preparation of pharmaceutical compositions in solid form which have consequently limited its clinical use as an active pharmacological ingredient.

Pharmaceutical compositions containing ursodeoxycholic acid in solid form for oral administration (in capsules or tablets), are administered with difficulty to children or to patients with deglutition difficulties. The solid forms currently available on the market are capsules or tables with a dosage of 150-300 mg of fast-release UDCA and 225-450 controlled-release UDCA. With this type of dosage it is difficult to dose with precision, to paediatric patients, the oral solid pharmaceutical forms according to body weight without making important posology errors. Capsules cannot be subdivided and tablets are not easily divisible into parts smaller than half (1/3, ¼ tablet) according to the patient's body weight.

In JP55022616 inclusion compounds of beta cyclodextrin with cholic acids were prepared. Pharmaceutical compositons for injection containing these inclusion compounds were described.

In the past, some attempts to solubilise UDCA, involving the use of various methods, have been made. The solubilisation methods traditionally employed for organic molecules have also been experimented for ursodeoxycholic acid.

The first method consists of the formation of salts, generally sodium. However, the sodium salts of ursodeoxycholic acid, when heated during certain phases of pharmaceutical preparation, such as ampoule sterilisation, tend to precipitate during the cooling phase.

The second method consists of the addition of surfactants to the UDCA sodium salt. In this case, solubilisation is obtained only at high pH values (9.5-11). Moreover, it is possible to observe either the formation of bubbles in the solution during the heating phase or to the decomposition of surface-active substances at high temperature with the loss of their activity.

The third methodology consists of the use of organic solvents such as benzylic alcohol. However, due to their high toxicity, Italian Health Authorities have long since prohibited the employment of organic solvents in the preparations of pharmaceutical compositions.

Lastly, a method has been tested which provides for the formation of a soluble ester of the UDCA active ingredient. This kind of approach was originally exploited for the preparation of esters of some antibiotics. To improve absorption of the antibiotics belonging to the beta-lactamine group (ampicillin, cephalexin and others), the esters of the active ingredient were synthesised. Active ingredients in the form of esters give rise to pharmaceutical compositions in solid form. The stability of these esters is considerably reduced in solution. Because of the reduced stability, active ingredients in the form of esters break up more easily already at the stomach level with a noticeable reduction in the availability of active ingredient.

The lack of availability of a soluble form of the active ingredient UDCA to be used to prepare pharmaceutical compositions in liquid form for oral or injectable use has practically precluded the employment of the active ingredient itself with some categories of patients such as paediatric patients and elderly patients. In particular a liquid pharmaceutical form of the UDCA active ingredient made soluble is essential in patients with limited intestinal absorption capacity or those with severe liver ailments such as active chronic hepatitis, cholestatic ailments of the adult and child such as primitive biliary cirrhosis, cholestasis induced by cyclosporin or associated to parenteral nutrition, cholestasis in the course of cystic fibrosis.

Therefore, the need remains to have available a method for rendering the UDCA active ingredient in soluble form, without the drawbacks of the prior art.

In particular, the need remains to have available soluble forms of the active ingredient UDCA to be used for the preparation of pharmaceutical compositions in liquid form.

In other words, pharmaceutical compositions are desirable in liquid form, including UDCA made soluble, for oral use or injectable to be destined also to patients to whom solid pharmaceutical forms cannot be administered.

One of the aims of the present invention is to provide a method for rendering the UDCA active ingredient in soluble form.

Another aim of the present invention is to provide a method for the preparation of pharmaceutical compositions in liquid form, which include UDCA in soluble form.

Yet a further aim of the present invention is to provide pharmaceutical compositions in liquid form, including UDCA in soluble form, for oral use or injectable.

These aims and others besides, which shall become more readily apparent from the description that follows, have been achieved by the Applicant, who has found it useful to render UDCA in soluble form through the formation of inclusion complexes with at least a binder.

The aims set out above and others besides which will become more readily apparent in the course of the description that follows are substantially achieved by a method, pharmaceutical compositions and their use as defined in the accompanying claims.

The subject of the invention is a method for rendering the ursodeoxycholic acid (UDCA) active ingredient in soluble form through the use of at least a binder belonging to the oligosaccharide class. In particular, the use of at least a binder of appropriate dimensions and polarity allows to obtain an inclusion complex through the "host-guest" integration (active ingredient-binder molecule). The binder molecules employed in the present invention can be chosen from the cyclodextrin class; preferably, α-cyclodextrins, β-cyclodextrins or γ-cyclodextrins are used; still more preferably, β-cyclodextrins are used.

β-cyclodextrin (abbreviated as CD) is a cyclic oligosaccharide derived from the enzymatic hydrolysis of common starch. β-cyclodextrin has the capability of forming, thanks to its particular cyclic structure, inclusion complexes with other molecules, of appropriate dimensions and polarity, increasing their degree of solubility in water and their bio-availability.

A further subject of the present invention further is constituted by the pharmaceutical compositions including ursodeoxycholic acid rendered in soluble form through the employment of a binder, chosen for instance from cyclodextrins, by the formation of soluble inclusion complexes.

Ursodeoxycholic acid (UDCA) and β-cyclodextrin (CD) tend to form hydrosoluble inclusion complexes. In particular, a preferred embodiment of the present invention consists of pharmaceutical compositions including ursodeoxycholic acid rendered soluble in water through the formation of hydrosoluble inclusion complexes in 1:2 molar ratio. The complex can be prepared by making the active ingredient and the binder react in aqueous solution at a temperature slightly lower than boiling temperature and neutralising the pH of the solution thereby obtained.

In a preferred embodiment, ursodeoxycholic acid is rendered soluble by means of the addition of β-cyclodextrin through the chemical synthesis of a molecular complex constituted by two β-cyclodextrin binder molecules and a molecule of ursodeoxycholic acid. Ursodeoxycholic acid can be considered as a bi-functional compound, from the standpoint of the formation of a complex with β-cyclodextrin, since an extremity of the molecule can be exclusively incorporated in the cavity of a single molecule of β-cyclodextrin (CD), and the other extremity is incorporated in another molecule of β-cyclodextrin (CD).

Preferably, the process of solubilisation of UDCA in the presence or β-cyclodextrin, described in the present invention, is performed starting from a concentrated aqueous suspension of the two powders in 1:2 molar ratio, respectively, and heating the suspension under continuous agitation until reaching near-boiling temperatures. Once a temperature ranging between 75°C and 90°C is reached, more preferably ranging between 80°C and 86°C, the complete clarification of the suspension is obtained, by effect of the inclusion reaction of ursodeoxycholic acid in the two β-cyclodextrin molecules which forms a water-soluble complex and the solution thus obtained is maintained under agitation at the temperature ranging between 75°C and 90°C for a period of time ranging between 5 and 20 minutes, more preferably between 10 and 15 minutes. Once this time interval has elapsed, the neutralisation of the pH of the solution is effected. The pH of the solution at the time of the reaction ranges between 3 and 4. Preferably the pH of the solution at the time of the reaction can range between 3.2 and 3.6. If the pH is not neutralised, once cooling is complete, the complex will precipitate, since its solubility in water is proportional to the pH. The pH of the solution is neutralised between values ranging from 6 to 8. Preferably, the pH of the solution is neutralised between values ranging from 6.5 to 7.5. The yield of the reaction can be estimated in at least 85%. Excellent reactions can lead to obtain yields that can be estimated in at least 90%.

The inclusion complex (1 ursodeoxycholic acid:2 β-cyclodextrin) can be used for preparing pharmaceutical compositions in any pharmaceutically acceptable form. Preferably, pharmaceutical compositions are in liquid form such as the syrup form and the injectable form, having succeeded in conveying ursodeoxycholic acid in aqueous solution.

Some details of the invention shall become more readily apparent from the description set out in the following examples:

### EXAMPLE 1

### (Preparation of inclusion complex)

11.35g (10mmoles) of β-cyclodextrin (CD) and 1.92 g of ursodeoxycholic acid (UDCA) (5mmoles) are placed in a 50 ml beaker of Pyrex glass. At least 12-15 ml of water and a magnetic armature are added, and the beaker is heated in a water bath and under vigorous agitation over a magnetic agitator with heating plate. The suspension has a milky appearance and its pH is about 3.5. Once the temperature of 83°C is reached, the suspension is clarified and the solution thus obtained is maintained under agitation at the temperature of 85°C for at least 10 minutes. After 10 minutes have elapsed, the pH is neutralised and the solution is dried in an oven at a temperature of 80°C. At the end of the drying operation, a crystalline white powder is obtained.

### EXAMPLE 2

### (Syrup formulation)

The inclusion complex (1 ursodeoxycholic acid:2 β-cyclodextrin) 41.47g is dissolved in water and with the addition of appropriate sweeteners such as sorbitol in powder form or already dissolved in water, aspartame, flavourings and flavour-correctors, were brought to 200 ml in order to have an overall dosage of 300 mg of UDCA per 5 ml.

### EXAMPLE 3

### (Syrup formulation)

The inclusion complex (1ursodeoxycholic acid:2 β-cyclodextrin) 20.735 g is dissolved in water and with the addition of appropriate sweeteners, flavourings and flavour-correctors, were brought to 200 ml in order to have an overall dosage of 150 mg of UDCA per 5 ml.

### EXAMPLE 4

### (Injectable formulation)

The inclusion complex (1 ursodeoxycholic acid:2 β-cyclodextrin) 11.82 g is dissolved in a litre of water of injectable solutions in order to prepare an intravenous solution equal to 2mg/ml of UDCA.

### Demonstration of the synthesis reaction by means of nuclear magnetic resonance

The inclusion complex (lursodeoxycholic acid:2 β-cyclodextrin), obtained according to the method of the present invention, was examined by means of protonic NMR spectroscopy at the Chemistry Department of "La Sapienza" University in Rome.

The bands of ursodeoxycholic acid were assigned based on the comparison with the data of the literature (J.Lipid. Red. 26 1068-1078 1985) (literature spectra executed with deuterated methanol). The complex was examined by means of TOCSY and ROESY two-dimensional NMR spectroscopy.

The TOCSY experiment enables to clarify constitutional aspects, since it is able to highlight interactions through the bonds present in individual molecules (confirmation of the structure of individual molecules). After obtaining this confirmation, the ROESY experiment was conducted, in order to highlight both the interactions through space present inside the molecules constituting the complex, and the interaction - also through space - between the chemical groups of the two constituents of the complex.

The presence of these contacts is proof of the interactions between the molecule of ursodeoxycholic acid and of β-cyclodextrin.

Magnetic resonances through such an experiment are able to highlight interactions at a distance of up to 5 10⁻¹⁰ m (Å) between functional groups belonging to different molecules.

The exam of the complex by means of the ROESY experiment has highlighted interactions of this type between the resonances related to ursodeoxycholic acid and the resonances of β-cyclodextrin.

The presence of such contacts, some of which were comparable in size to intra-molecular effects, is extremely indicative of (short range) interactions between different groups of the two different molecules and consequently of the presence of the complex.

Spectra were executed in 99.96% D₂O solution dissolving 5 mg of the complex in 5 ml.

A further subject of the present invention is given by the use of the ursodeoxycholic acid made available through the formation of inclusion complexes for the preparation of pharmaceutical compositions to be destined to patients for preventing the formation of cholesterol biliary stones or to facilitate their dissolution if they are already formed.

Administered to humans in oral liquid form, the UDCA made soluble is immediately available at the intestinal level, facilitating absorption. β-cyclodextrin does not overcome the barrier of the intestinal mucosa and is not found either in plasma or in urine. β-cyclodextrin is metabolised in the colon by the bacterial micro-flora, producing linear dextrins, maltose and glucose.

The advantages of the inclusion complex (lursodeoxycholic acid:2 β-cyclodextrin) in aqueous solution are pharmaceutical and clinical in nature:
- ursodeoxycholic acid, subjected to the process of the present invention, is highly soluble, and therefore it can be used in the preparation of oral liquid formulations (syrup, drops, granules for extemporaneous solutions) and injectable forms;
- UDCA, made soluble in water with the method of the present invention, is characterised by high stability, so it can be used for the preparation of medication and can be preserved under ordinary conditions of temperature, humidity and light, without losing its effectiveness over time. In this regard, the following stability tests were conducted.
   TEST 1 - an aqueous solution of the complex at the concentration of 11.82 mg/ml was placed in colourless glass bottles sealed with screw-on cap and left under normal temperature and light conditions for six months. The solution showed no turbidity and chemical analysis showed no signs of degradation.
   TEST 2 - an aqueous solution of the complex at the concentration of 23.64 mg/ml, obtained as in example 1, was placed in a thermostatically-controlled stove at 40°C for a six-month interval. Analyses were conducted to identify the chemical-physical characteristics and no turbidity or degradation by-products were detected.
- the bio-availability of UDCA, made soluble in water by the addition of β-cyclodextrin, is increased: after administering the complex in solution, higher AUC and concentration peaks (Cmax) are observed than for oral solid forms; maximum peak time (Tmax) was found to be shorter. Consequently, for the same dosage, the administration of UDCA solubilised by means of β-cyclodextrin has the advantage of guaranteeing a higher quantity of drug available for therapeutic action;
- β-cyclodextrin has no toxicity and it is allowed by FAO/WHO as an additive for the preparation of foodstuffs;
- UDCA, made soluble in water by the addition of β-cyclodextrin, finds easy application for oral administration in the paediatric age, in patients with deglutition difficulties (elderly); it can also be used for the preparation of solutions to be injected parenterally which allow the employment of UDCA in pathologies characterised by reduced absorption of the drug, in particular in intra-hepatic cholestatic forms of adults and children.

The Applicant has conducted a comparison test between the absorption of UDCA derived from the inclusion complex (lursodeoxycholic acid:2 β-cyclodextrin) in solution and UDCA derived from a quick-release solid form currently on the market (300 mg capsules).

The administered dose was, for both forms, 300 mg; the test was conducted on 5 adult subjects, healthy and fasting.

To check response variability, a paired sample design was adopted, i.e. one in which each subject assumed both formulations in two distinct sessions separated by a 7-day interval. This interval is sufficient for the drug assumed during the first session to be fully eliminated before assumption of the second one. In this way, interaction phenomena are avoided and the results of the two sessions are to be considered independent.

The serum levels of UDCA were determined spectro-photometrically with an appropriate kit acquired for the purpose.

Drawing times were defined based on the properties of UDCA, whose rapid and complete absorption at the intestinal level is well known. The first 4 points are closer than the others because a more rapid absorption of soluble UDCA than for traditional formulations was expected.

The results of the UDCA serum levels are shown in the accompanying Plate 1 (table), where pmoll/litre concentrations obtained after converting the data measured with the spectro-photometer in O.D. (Optical Density) are highlighted with a black circle.

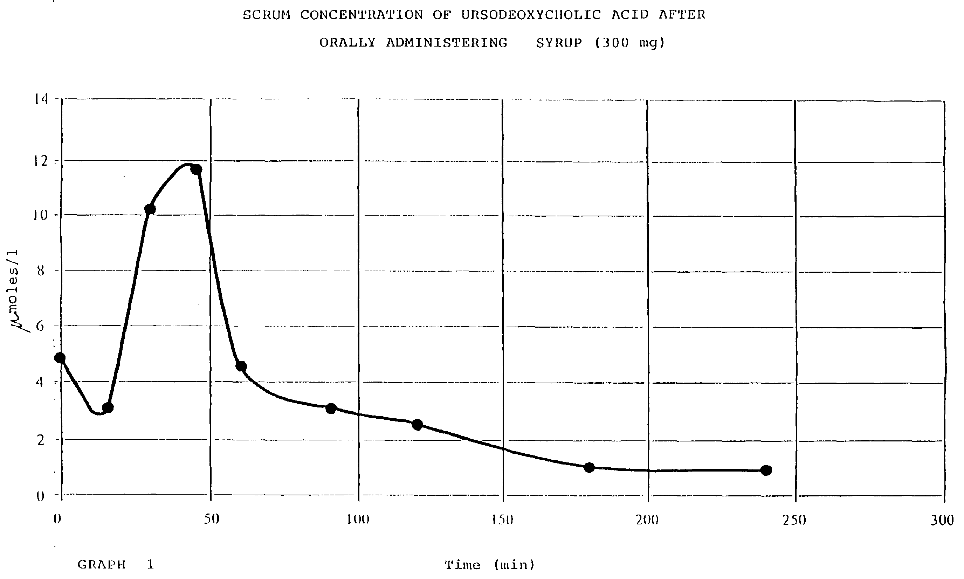

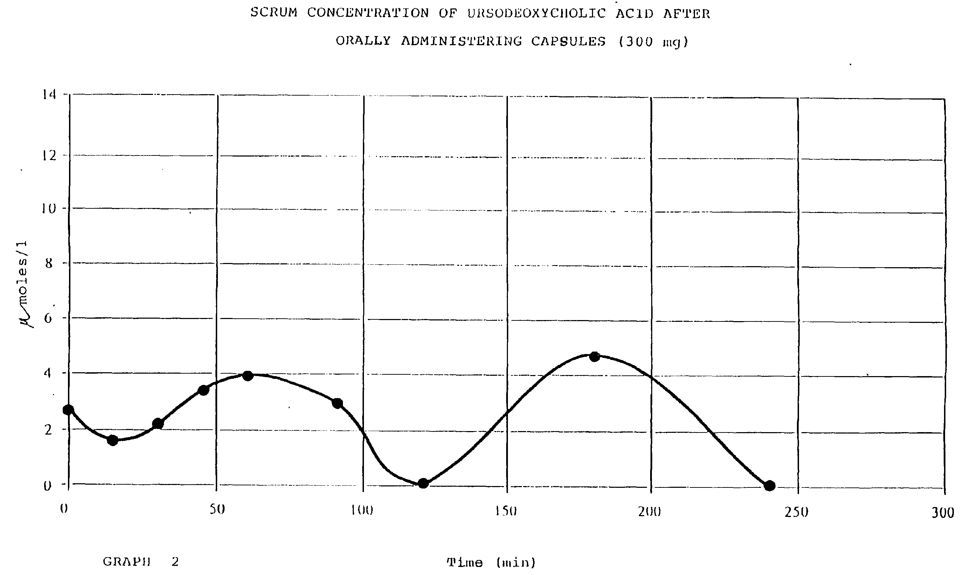

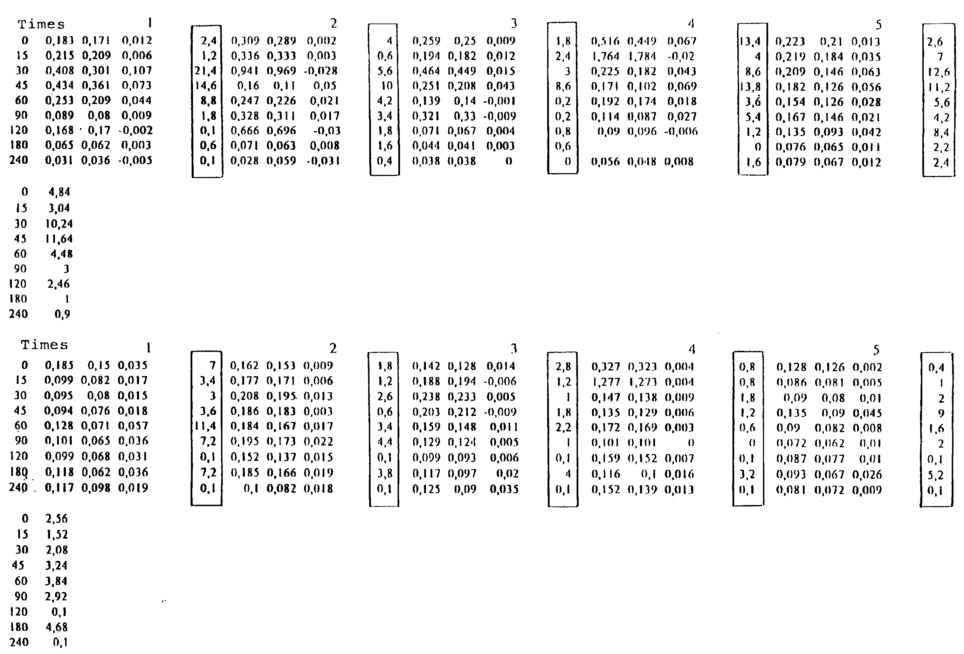

The effect of solubilisation on UDCA absorption kinetics is demonstrated by the data shown in graphic form in the accompanying Plate 2 (Graph 1) and in Plate 3 (Graph 2). The concentration/time curve of mean values for UDCA from UDCA-β-cyclodextrin in soluble form has a precocious and high peak (Cmax=11.8 and Tmax=45 min). AUC computed with the trapezoid rule is equal to 778, see Plate 2 (Graph 1).

By contrast, the profile of the curve after 300 mg of UDCA in quick-release capsule is different, appearing flat with respect to the previous one and bimodal, expressing a re-circulation phenomenon, with AUC=697, i.e. -11% off the previous one. Cmax is lower and Tmax is delayed, but these measurements have only a relative meaning due to the dual peak noted, see Plate 3 (Graph 2).

The comparison allows to derive the specific characteristics of the absorption UDCA-β-cyclodextrin which show, for the same dose as the solid form available on the market, that availability is 12% greater and absorption time is shorter.

Relative bio-availability, for the same dose and constant total clearance, is equal to 1.11.

## Claims

1. A method for preparing inclusion complexes in soluble form of ursodeoxycholic acid with cyclodextrins, comprising the following steps:
a) preparing an aqueous suspension of ursodeoxycholic acid and a binder selected among cyclodextrins;
b) heating the suspension deriving from step a) until obtaining a clear solution;
**characterized in that**:
c) the acid pH of the solution deriving from step b) is neutralised to values ranging from 6.5 to 7.5;
d) the solution deriving from step c) is dried until obtaining a crystalline powder soluble in water, constituted by inclusion complexes of ursodeoxycholic acid with said cyclodextrin binder.

2. The method as claimed in claim 1, wherein the ursodeoxycholic acid and said cyclodextrin binder form inclusion complexes in 1:2 molar ratio.

3. The method as claimed in claim 2, wherein the binder is selected among α-cyclodextrins.

4. The method as claimed in claim 2, wherein the binder is selected among β-cyclodextrins.

5. The method as claimed in claim 2, wherein the binder is selected among γ-cyclodextrins.

6. The method as claimed in claim 1, wherein, in said step a), the ursodeoxycholic acid and said binder are suspended in water in 1:2 molar ratio.

7. The method as claimed in claim 1, wherein, in said step b), the suspension is brought to a temperature ranging between 80° and 90° C.

8. The method as claimed in claim 1, wherein, in said step d), the drying temperature is ranging between 70° and 90°C.

## Patentansprüche

1. Verfahren zur Herstellung Einschlusskomplexe in löslicher Form von Ursodeoxycholsäure mit Cyclodextrinen, das die folgenden Schritte umfasst:
a) Herstellung einer wässrigen Suspension aus Ursodeoxycholsäure und einem Bindemittel, das unter Cyclodextrinen ausgewählt ist;
b) Erwärmung der Suspension aus Schritt a) bis zum Erhalten einer klaren Lösung;
**dadurch gekennzeichnet, dass**
c) der saure pH-Wert der Lösung aus Schritt b) auf Werte im Bereich von 6,5 bis 7,5. neutralisiert wird;
d) die Lösung aus Schritt c) bis zum Erhalt eines wasserlöslichen kristallinen Pulvers getrocknet wird, das aus Einschlusskomplexen von Ursodeoxycholsäure mit dem genannten Cyclodextrin-Bindemittel besteht.

2. Verfahren nach Anspruch 1, wobei die Ursodeoxycholsäure und das genannte Cyclodextrin-Bindemittel Einschlusskomplexe im Molverhältnis von 1:2 bilden.

3. Verfahren nach Anspruch 2, wobei das Bindemittel unter α-Cyclodextrinen ausgewählt ist.

4. Verfahren nach Anspruch 2, wobei das Bindemittel unter β-Cyclodextrinen ausgewählt ist.

5. Verfahren nach Anspruch 2, wobei das Bindemittel unter γ-Cyclodextrinen ausgewählt ist.

6. Verfahren nach Anspruch 1, wobei im genannten Schritt a) die Ursodeoxychölsäure und das genannte Bindemittel im Molverhältnis von 1:2 in Wasser suspendiert werden.

7. Verfahren nach Anspruch 1, wobei im genannten Schritt b) die Suspension auf eine Temperatur im Bereich zwischen 80°C und 90°C gebracht wird.

8. Verfahren nach Anspruch 1, wobei im genannten Schritt d) die Trocknungstemperatur im Bereich zwischen 70°C und 90°C liegt.

## Revendications

1. Méthode pour l'obtention de complexes d'inclusion sous forme soluble d'acide ursodeoxycholique avec des cyclodextrines, comprenant les phases suivantes:
a) la préparation d'une suspension aqueuse d'acide ursodeoxycholique et un ligand choisi parmi des cyclodextrines;
b) le réchauffement de la suspension dérivant de la phase a) jusqu'à l'obtention d'une solution claire;
**caractérisée en ce que**
c) la valeur de pH acide de la solution dérivant de la phase b) est neutralisée à, des valeurs dans une plage de 6, 5 à 7,5;
d) la solution dérivant de la phase c) est desséchée jusqu'à l'obtention d'une poudre cristalline soluble dans l'eau, constituée par des complexes d'inclusion d'acide ursodeoxycholique avec ledit ligand de cyclodextrine.

2. Méthode selon la revendication 1, dans laquelle l'acide ursodeoxycholique et ledit ligand de cyclodextrine forment des complexes d'inclusion dans un rapport molaire de 1:2.

3. Méthode selon la revendication 2, dans laquelle le ligand est choisi parmi des α-cyclodextrines.

4. Méthode selon la revendication 2, dans laquelle le ligand est choisi parmi des β-cyclodextrines.

5. Méthode selon la revendication 2, dans laquelle le ligand est choisi parmi des γ-cyclodextrines.

6. Méthode selon la revendication 1, dans laquelle, dans ladite phase a), l'acide ursodeoxycholique et ledit ligand sont suspendus dans l'eau dans un rapport molaire de 1:2.

7. Méthode selon la revendication 1, dans laquelle, dans ladite phase b), la suspension est portée à une température dans une plage de 80°C à 90°C.

8. Méthode selon la revendication 1, dans laquelle, dans ladite phase d), la température de dessiccation est dans une plage de 70°C à 90°C.
